# EUROPEAN PATENT SPECIFICATION

(11) **EP 4 193 991 B1**
(45) Date of publication and mention of the grant of the patent: **14.05.2025**
(21) Application number: 21853332.1
(22) Date of filing: 26.07.2021
(51) Int. Cl.: A61K 9/50, A61K 9/26, A61K 31/551, A61P 35/00, A61K 9/20

(54) **ORBITAZINE FUMARATE ENTERIC-COATED PELLET, PREPARATION METHOD THEREFOR AND USE THEREOF**
ENTERISCH BESCHICHTETES ORBITAZINFUMARATPELLET, HERSTELLUNGSVERFAHREN DAFÜR UND VERWENDUNG DAVON
GRANULE ENROBÉE ENTÉRIQUE DE FUMARATE D'ORBITAZINE, SON PROCÉDÉ DE PRÉPARATION ET SON UTILISATION

(30) Priority: 07.08.2020 CN 202010789585
(43) Date of publication of application: 14.06.2023
(73) Proprietor: SHENZHEN ZHENXING MEDICAL TECHNOLOGY CO., LTD., Business Secretary Co., Ltd.) Guangdong 518000 (CN)
(72) Inventor: CHENG, Liren, Hong Kong Cooperation Zone (get Settled In Shenzhen Qianhai Business Secretary Co., Ltd.) SHENZHEN, Guangdong 518000 (CN)
(74) Representative: Herrero & Asociados, S.L.
(86) International application number: PCT/CN2021/108360
(87) International publication number: WO 2022/028264

(56) References cited:
- WO-A1-2010/102513
- CN-A- 101 502 517
- CN-A- 101 503 394
- CN-A- 105 085 421
- CN-A- 111 743 876
- LIU SHUJIE, YU MIN;WANG YU;HUANG SHU-JIA;DAN MO;LI ZUO-GANG;GENG XING-CHAO;ZHANG HE-ZHAN;YANG JIN-BO;LIU LI: "Establishment and validation of UPLC － MS / MS method for determination of an innovative antitumor drug SM － 1 in plasma of Beagle dogs", CHINESE JOURNAL OF NEW DRUGS, GAI-KAN BIANJIBU, BEIJING, CN, vol. 29, no. 12, 30 June 2020 (2020-06-30), CN , pages 1349 - 1354, XP055895473, ISSN: 1003-3734

## Description

This application is based on and claims priority to CN Patent Application No. 202010789585.5 filed August 7, 2020. which application is incorporated herein by reference in its entirety.

### FIELD OF THE INVENTION

The invention relates to the field of pharmaceutical chemistry, in particular to orbitazine fumarate enteric-coated pellets, and preparation therefor and use thereof in manufacture of a medication for the prevention and/or treatment of tumors.

### BACKGROND OF THE INVENTION

Orbitazine fumarate, with a chemical name of (4-benzyl - [1,4]diazocycloheptan-1-yl) - acetyl (3-allyl-2-hydroxy-methylenephenyl) hydrazine fumarate, has a chemical formula of:

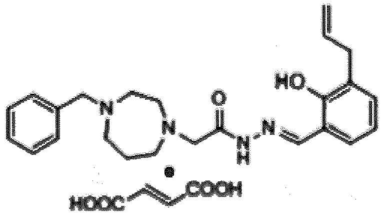

WO2010102513A1 disclosed the structure, preparation and use of orbitazine fumarate and its derivatives, which can specifically activate procasapase-3 in tumor cells to active caspase-3, and thus induce tumor cell apoptosis. This document also further disclosed that these compounds have significant inhibitory effects on the growth of various cancer cell lines, such as HL-60 cells, NCI-H460 cells, HepG2 cells, and A549 cells. As the expression of procaspase-3 in tumor cells is much higher than that in normal cells, orbitazine fumarate is expected to become a new anti-tumor drug regulating apoptosis signal transduction pathway.

### SUMMARY OF THE INVENTION

The inventor of this application has conducted extensive researches on the formulation and preparation of orbitazine fumarate, and developed an enteric coated pellets preparation of orbitazine fumarate. The excipients used in this preparation have good compatibility with orbitazine fumarate, and the preparation showed good stability, high safety, rapid disintegration and stable release in intestinal fluid, and thus the bioavailability of orbitazine fumarate are enhanced. The enteric coated pellets are prepared by extrusion spheronization process, which is conducive to expanding the drug loading range of the pellets, facilitating the adjustment of the dosage specifications of the pellets according to clinical needs, and realizing industrial production, with short time consumption and good effect.

The invention is defined in the claims.

In the first aspect, the invention provides enteric coated pellets of orbitazine fumarate; in the second aspect, the invention provides a method for preparation of the enteric coated pellets; in the third aspect, the invention provides the enteric coated pellets for use in the treatment of tumors.

Specifically, in the first aspect, the invention provides an enteric coated pellet of orbitazine fumarate, which comprises a) a pellet core containing orbitazine fumarate, b) an isolation layer and c) an enteric layer, wherein the enteric layer comprises an enteric coating material, which is hydroxypropyl methylcellulose acetate succinate or hydroxypropyl methylcellulose phthalate.

In some embodiments, the pellet core also comprises a diluent, disintegrant and cosolvent.

In other embodiments, the diluent is selected from one or more of microcrystalline cellulose, lactose and pregelatinized starch.

In other embodiments, the diluent is microcrystalline cellulose.

In other embodiments, the diluent is lactose.

In other embodiments, the diluent is pregelatinized starch.

In other embodiments, the disintegrant is selected from one or more of sodium carboxymethyl starch, low substituted hydroxypropyl cellulose, cross-linked sodium carboxymethyl cellulose, and cross-linked povidone.

In other embodiments, the cosolvent is selected from one or more of polyvinylpyrrolidone, Tween-20, Tween-60, Tween-80, and sodium dodecyl sulfate.

In other embodiments, the cosolvent is polyvinylpyrrolidone.

In other embodiments, the cosolvent is Tween-20.

In other embodiments, the cosolvent is Tween-60.

In other embodiments, the cosolvent is Tween-80.

In other embodiments, the cosolvent is sodium dodecyl sulfate.

In other embodiments, the cosolvent is a combination of polyvinylpyrrolidone and Tween-20.

In some embodiments, the diluent in the pellet core is selected from one or more of microcrystalline cellulose, lactose, and pregelatinized starch; the disintegrant is selected from one or more of sodium carboxymethyl starch, cross-linked sodium carboxymethyl cellulose, and cross-linked povidone; and the cosolvent is selected from one or more of polyvinylpyrrolidone, Tween-20, Tween-60, Tween-80, and sodium dodecyl sulfate.

In other embodiments, the disintegrant is:
sodium carboxymethyl starch,
cross-linked sodium carboxymethyl cellulose,
cross-linked povidone,
combination of low substituted hydroxypropyl cellulose and sodium carboxymethyl starch,
combination of low substituted hydroxypropyl cellulose and cross-linked sodium carboxymethyl cellulose, or
combination of low substituted hydroxypropyl cellulose and cross-linked povidone.

In other embodiments, the disintegrant is sodium carboxymethyl starch.

In other embodiments, the disintegrant is cross-linked sodium carboxymethyl cellulose.

In other embodiments, the disintegrant is cross-linked povidone.

In other embodiments, the disintegrant is a combination of low substituted hydroxypropyl cellulose and sodium carboxymethyl starch.

In other embodiments, in the combination of low substituted hydroxypropyl cellulose and sodium carboxymethyl starch, the mass ratio of the low substituted hydroxypropyl cellulose to sodium carboxymethyl starch is 0.1-3.5:1, such as 0.5:1, 1:1, 1.4:1, 1.5:1, 1.75:1, 2:1, 2.3:1, 2.5:1 or 3:1.

In other embodiments, the disintegrant is a combination of low substituted hydroxypropyl cellulose and cross-linked sodium carboxymethyl cellulose.

In other embodiments, in the combination of low substituted hydroxypropyl cellulose and cross-linked sodium carboxymethyl cellulose, the mass ratio of the low substituted hydroxypropyl cellulose and the cross-linked sodium carboxymethyl cellulose is 0.1-3.5:1, such as 0.5:1, 1:1, 1.4:1, 1.5:1, 1.75:1, 2:1, 2.3:1, 2.5:1 or 3:1.

In other embodiments, the disintegrant is a combination of low substituted hydroxypropyl cellulose and cross-linked povidone.

In other embodiments, in the combination of low substituted hydroxypropyl cellulose and cross-linked povidone, the mass ratio of the low substituted hydroxypropyl cellulose and the cross-linked povidone is 0.1-3.5:1, such as 0.5:1, 1:1, 1.4:1, 1.5:1, 1.75:1, 2:1, 2.3:1, 2.5:1 or 3:1.

In other embodiments, the pellet core comprises, by weight:
Orbitazine fumarate, 10-30 portions;
Microcrystalline cellulose, 4-12 portions;
Sodium carboxymethyl starch, 0-5 portions;
Low substituted hydroxypropyl cellulose, 2-8 portions
Tween, 0-4 portions;
Polyvinylpyrrolidone, 0-3 portions.

In other embodiments, the pellet core comprises, by weight:
Orbitazine fumarate, 12-18 portions;
Microcrystalline cellulose, 6-10 portions;
Sodium carboxymethyl starch, 1-4 portions;
Low substituted hydroxypropyl cellulose, 3-5 portions
Tween, 0.5-3 portions;
Polyvinylpyrrolidone, 0.5-2 portions.

In other embodiments, the pellet core comprises, by weight:
Orbitazine fumarate, 14-16 portions;
Microcrystalline cellulose, 8-9 portions;
Sodium carboxymethyl starch, 2-3 portions;
Low substituted hydroxypropyl cellulose, 4-5 portions
Tween, 1-2 portions;
Polyvinylpyrrolidone, 0.8-1.5 portions.

In some embodiments, the isolation layer comprises isolation materials,

optionally, the isolation layer comprises also an anti-adhesive.

In some embodiments, the isolation material is one or two of hydroxypropyl methylcellulose and hydroxypropyl cellulose.

In some embodiments, the isolation material is hydroxypropyl methyl cellulose.

In some embodiments, the isolation material is hydroxypropyl cellulose.

In some embodiments, the isolation material is a combination of hydroxypropyl methyl cellulose and hydroxypropyl cellulose.

In some embodiments, the anti-adhesive in the isolation layer is talcum powder.

In some embodiments, the enteric layer comprises an enteric coating material,

Optionally, the enteric coating also includes a plasticizer and/or an anti-adhesive.

In some embodiments, the enteric coating material is selected from one or more of hydroxypropyl methylcellulose acetate succinate and hydroxypropyl methylcellulose phthalate.

In some embodiments, the enteric coating material is hydroxypropyl methylcellulose acetate

In some embodiments, the enteric coating material is hydroxypropylmethylcellulose phthalate.

In some embodiments, the plasticizer is triethyl citrate.

In some embodiments, the anti-adhesive in the enteric layer is talcum powder or glyceryl monostearate or a combination thereof.

In some embodiments, the anti-adhesive in the enteric layer is talcum powder.

In some embodiments, the anti-adhesive in the enteric layer is glyceryl monostearate.

In some embodiments, the anti-adhesive in the enteric layer is a combination of talcum powder and glyceryl monostearate.

In some embodiments, the enteric coated pellet comprises: a) a pellet core containing orbitazine fumarate, b) an isolation layer, and c) an enteric coated layer, with the mass percentage of them being as follows:
a) pellet core 60%-78%;
b) isolation layer 10%-20%, and
c) enteric layer 12%-20%.

In other embodiments, a pellet core containing orbitazine fumarate, b) an isolation layer, and c) an enteric coated layer, with the mass percentage of them being as follows:
a) pellet core 62%-72%;
b) isolation layer 14%-20%, and
c) enteric layer 14%-18%.

In some embodiments, the weight of the isolation layer of the enteric coated pellet is 10%-20% of the weight of the pellet core containing orbitazine fumarate.

In some embodiments, the weight of the isolation layer of the enteric coated pellet is 14%-20% of the weight of the pellet core containing orbitazine fumarate.

In some embodiments, the weight of the isolation layer of the enteric coated pellet is 14%-18% of the weight of the pellet core containing orbitazine fumarate.

In some embodiments, the weight of the isolation layer of the enteric coated pellet is 14%-16% of the weight of the pellet core containing orbitazine fumarate.

In some embodiments, the weight of the enteric layer of the enteric coated pellet is 16%-26% of the weight of the pellet core containing orbitazine fumarate.

In some embodiments, the weight of the enteric layer of the enteric coated pellet is 16%-20% of the weight of the pellet core containing orbitazine fumarate.

In some embodiments, the weight of the enteric layer of the enteric coated pellet is 17%-20% of the weight of the pellet core containing orbitazine fumarate.

In some embodiments, the weight of the enteric layer of the enteric coated pellet is 18%-20% of the weight of the pellet core containing orbitazine fumarate.

In some embodiments, the enteric coated pellet comprises, by weight:
Orbitazine fumarate, 10-30 portions;
Microcrystalline cellulose, 4-12 portions;
Sodium carboxymethyl starch, 0-5 portions;
Low substituted hydroxypropyl cellulose, 2-8 portions
Tween, 0-4 portions;
Polyvinylpyrrolidone, 0-3 portions;
Hydroxypropyl methyl cellulose, 1-5 portions;
Hydroxypropyl methylcellulose acetate succinate, 1-5 portions;
Triethyl citrate, 0-3 portions;
Glyceryl monostearate, 0-0.5 portions; and
Talcum powder, 0-5 portions.

In some embodiments, the enteric coated pellet comprises, by weight:
Orbitazine fumarate, 14-16 portions;
Microcrystalline cellulose, 8-9 portions;
Sodium carboxymethyl starch, 2-3 portions;
Low substituted hydroxypropyl cellulose, 4-5 portions
Tween, 1-2 portions;
Polyvinylpyrrolidone, 0.8-1.5 portions;
Hydroxypropyl methyl cellulose, 2.5-3.5 portions;
Hydroxypropyl methylcellulose acetate succinate, 2.5-3.5 portions;
Triethyl citrate, 0.5-1.5 portions;
Glyceryl monostearate, 0.08-0.15 portions; and
Talcum powder, 1.5-2 portions.

In some embodiments, the pellet size of the enteric coated pellet is between 0.3-1.5 mm.

In some embodiments, the orbitazine fumarate in the enteric coated pellet is of amorphous, crystalline form A or crystalline form B.

In some embodiments, the particle size D90 of orbitazine fumarate particles in the enteric coated pellet is less than or equal to 50 µm.

In other embodiments, the particle size D90 of orbitazine fumarate particles in the enteric coated pellet is less than or equal to 30 µm.

Further, the disclosure provides a capsule or tablet, which is encapsulated or tableted from the enteric coated pellet of any one of the preceding embodiments.

In other embodiments, the content of orbitazine fumarate in the capsule or tablet is 0-200mg.

In other embodiments, the content of orbitazine fumarate in the capsule or tablet is 5mg, 10mg, 25mg, 50mg, 100mg or 200mg.

In the second aspect, the invention provides a method for preparation of the enteric coated pellets, which method comprises the steps of:
a) mixing orbitazine fumarate with diluent and disintegrant to obtain a premix;
b) mixing the premix with the cosolvent to prepare a soft material, wherein the cosolvent is obtained by mixing the cosolvent with a 50%-70% (preferably 55%-65%, such as 60%) ethanol aqueous solution;
c) the soft material is extruded, rounded, pelleted and dried;
d) wrapping with an isolation layer;
e) wrapping with an enteric layer.

In the third aspect, the invention provides the enteric coated pellet for use in the treatment of tumors.

In some embodiments, the tumors are acute promyelocytic leukemia, large cell lung cancer, liver cancer or non-small cell lung cancer.

### Details of the Invention:

Before describing the invention in more detail, it should be understood that the invention is not limited to the specific embodiments described herein, because such embodiments can be modified or changed. It should also be understood that the terms used herein are for the purpose of describing specific embodiments only, and are not used for limitation. Unless otherwise specified, all technical and scientific terms used herein have the same meanings generally understood by those skilled in the art.

The terms "mass percentage", "weight percentage" or "percentage by weight" or "wt%" are defined as the weight of a single component in a preparation divided by the total weight of all components in the preparation and multiplied by 100%. In the invention, "%" refers to the mass percentage unless otherwise specified.

In the description presented above, all figures disclosed herein are approximate regardless of whether the word "approximately" or "about" is used. The value of each figure may have a difference of less than 10% or a reasonable difference considered by a person of skills in the art, such as 1%, 2%, 3%, 4% or 5%.

The term "D₉₀" refers to the particle size corresponding to 90% of the cumulative particle size distribution of a sample. Its physical meaning is that 90% of the particles are smaller than it, for example, "D₉₀ is less than or equal to 50 µM " means that the particles with a particle size is not greater than 50µm account for 90.

The term " AUC_{0-∞}" refers to the area under the concentration time curve (AUC) extrapolated to infinity or AUC+ (the last measured concentration/elimination rate constant) to the time point of the last measurement.

The term " Cₘₐₓ " is defined as the maximum plasma concentration of the active ingredient measured.

The preparation provided by the invention can be given to patients alone, or given together or in combination with other active preparations. The terms "co-administration" and "combination" include the simultaneous or sequential administration of two or more therapeutic agents without a specific time limit. In one embodiment, the reagent simultaneously exists in cells or individuals, or simultaneously exerts biological or therapeutic effects. In one embodiment, each of therapeutic agents is in the same composition or unit dosage form. In other embodiments, each of the therapeutic agents is in different composition or unit dosage form. In some embodiments, before administration of the second therapeutic agent (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks or 12 weeks ago), the first agent is given simultaneously or later (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks or 12 weeks later).

The term "diluent" includes but is not limited to microcrystalline cellulose, lactose, compressible sugar, dextrose, mannitol, dextrin, maltodextrin, sorbitol, xylitol, sodium chloride, calcium carbonate, magnesium carbonate, calcium phosphate, calcium sulfate, magnesium oxide, kaolin, powdered cellulose, pregelatinized starch, starch, barium sulfate, magnesium trisilicate, aluminum hydroxide and combinations thereof. In some embodiments, the diluent of the invention comprises at least microcrystalline cellulose. In other embodiments, the diluent of the invention includes microcrystalline cellulose and one or more of selected from mannitol, lactose, lactose monohydrate, pregelatinized starch, sorbitol, calcium hydrogen phosphate, starch and sucrose.

The term "disintegrant" includes but is not limited to corn starch, sodium carboxymethyl starch, low substituted hydroxypropyl cellulose, microcrystalline cellulose, cross-linked sodium carboxymethyl cellulose, cross-linked polyvinyl pyrrolidone (cross-linked povidone ,PVP), alginate, sodium alginate, guar gum, etc.

The term "cosolvent" includes but is not limited to sodium benzoate, citric acid, calcium lactate, ethanol, isooctanol, polyvinylpyrrolidone, Tween-20, Tween-60, Tween-80, sodium dodecyl sulfate, etc.

The beneficial effects of the invention include:
The enteric coated pellets containing orbitazine fumarate of the invention have good compatibility with orbitazine fumarate, good preparation stability, high safety, rapid disintegration and stable release in intestinal fluid, thus improving the bioavailability of orbitazine fumarate;
The enteric coated pellets are prepared by extrusion spheronization process, which is conducive to expanding the drug loading range of the pellets, facilitating the adjustment of the dosage specifications of the pellets according to clinical needs, and realizing industrial production, with short time consumption and good effect.

### BRIEF DESCRIPTION OF THE DRAWINGS

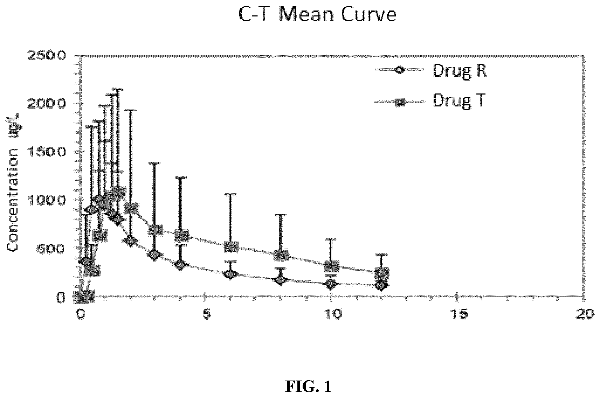

FIG. 1. Concentration-time curve (C-T curve) of a single dose of orbitazine in beagle dogs.

### DETAILED DESCRIPTION OF THE EMBODIMENTS

The following will further explain the substantive content of the invention in combination with the specific embodiments of the invention. It should be understood that the following embodiments are only used to explain the invention, but not to limit the protection scope of the invention. If the specific conditions are not indicated in the following examples, the general conditions or the manufacturer's recommendations shall be followed. The raw materials used without specifying the manufacturer are all conventional products that can be purchased in the market.

Although many materials and operation methods used in the following embodiments are well known in the art, some are still described herein as much detail as possible. It is clear to those skilled in the art that, unless otherwise specified, the materials and operation methods used in the following embodiments are well known in the art.

In the following embodiments: rpm refers to revolutions per minute; RRT refers to relative retention time; Min is minute; ND means no detection; DCPA refers to anhydrous calcium hydrogen phosphate; MCC PH200 is microcrystalline cellulose PH200; SDS means sodium dodecyl sulfate; HPMC-AS refers to hydroxypropyl methylcellulose acetate succinate; HPMCP refers to hydroxypropyl methylcellulose phthalate; API refers to orbitazine fumarate; SD refers to standard deviation.

The reagents used in the invention can be purchased from the market or prepared by the method described in the invention. Orbitazine fumarate was purchased from Dongguan Chang'an Dongyangguang Pharmaceutical Research and Development Co., Ltd., microcrystalline cellulose was purchased from Asahi Kasei, Japan, sodium carboxymethyl starch was purchased from Anhui Shanhe Auxiliary Materials Co., Ltd., low substituted hydroxypropyl cellulose was purchased from Anhui Shanhe Auxiliary Materials Co., Ltd., Tween 20/Tween 80 was purchased from Nanjing Weier Chemicals, Polyvinylpyrrolidone (k30) was purchased from Ashland, and pregelatinized starch was purchased from Rogate, Hydroxypropyl methylcellulose (E3) was purchased from Anhui Shanhe Auxiliary Materials Co., Ltd., talcum powder was purchased from Guangxi Longsheng Huamei Talc Development Co., Ltd., hydroxypropyl methylcellulose acetate (LG) was purchased from Ashland/Shinyue, Japan, triethyl citrate was purchased from Bengbu Fengyuan Tushan Pharmaceuticals Co., Ltd., and glycerol monostearate was purchased from Hunan Erkang Pharmaceuticals.

### EXAMPLES

### Example 1: Preparation and stability test of enteric coated pellets

**Table 1: Prescription List**

| Structure | Composition | Feeding amount (g) |
|---|---|---|
| | Orbitazine fumarate | 1125.10 |
| | Microcrystalline cellulose | 625.12 |
| Plain pellets | Sodium carboxymethyl starch | 200.20 |
| | Low substituted hydroxypropyl cellulose | 350.32 |
| | Tween 20 | 125.37 |
| | Polyvinylpyrrolidone (k30) | 75.12 |
| Isolation coating | Plain pellets | 2000.80 |
| | Hydroxypropyl methyl cellulose (E3) | 241.12 |
| | Talcum powder | 60.08 |
| | Coating solvent (purified water) | 5702.23 |
| Enteric coating | Insolated pellets | 2000.10 |
| | hydroxypropyl methylcellulose acetate succinate (LG) | 228.02 |
| | Talcum powder | 76.03 |
| | Triethyl citrate | 56.12 |
| | Glyceryl monostearate | 8.10 |
| | Purified water | 714.48 |
| | Anhydrous ethanol | 2857.80 |

### 1. Preparation of enteric coated pellets

First, pre-treatment: Orbitazine fumarate API passes through YK-60 equipment swing granulator (Changsha Yi Pharmaceutical Machinery Co., Ltd.), through 24 mesh screen, and is pretreated for standby;

Second, soft material mixing: 1125.10g of pretreated API, 625.12g of microcrystalline cellulose, 200.20g of sodium carboxymethyl starch and 350.32g of low substituted hydroxypropyl cellulose were added to a wet granulating machine (Shenzhen Xinyit Technology Co., Ltd.), and mixed for 5 minutes at the rotating speed of the stirring paddle of 1-3 rps and the cutting knife of 10-40 rps. Add a ethanol (60%, 900ml) solution of 75.12g of polyvinylpyrrolidone (k30) and 125.37g of Tween 20, set the speed of the stirring paddle to 1-3 rps, and the speed of the cutting knife to 20-60 rps, and stop mixing and cutting after adding.

Third, extrusion and spheronization: the soft materials prepared in the second step are extruded through 0.3-1.5 mm orifice plates with an extrusion and spheronization machine (Shenzhen Xinyit Technology Co., Ltd.), and then spheronized in the way of first high speed (600-1600 rpm) and then low speed (100-500 rpm). The high speed spheronization time and low speed spheronization time are adjusted according to the specific situation. During the spheronization process, an appropriate amount of 60% - 95% ethanol can be sprayed to help the pellet molding.

Fourth, drying: the pellets (wet materials) prepared by spheronization are dried in a fluidized bed (Shenzhen Xinyite Technology Co., Ltd.). The inlet air temperature is 45 ° C, the material temperature is controlled at 35-45 ° C, and the drying time is about 30min.

Fifth, isolation coating

Preparation of coating solution: Heat about 1/2 of the purified water with the prescription amount to about 60 ° C, stir it into a vortex, slowly add 241.12g of hydroxypropyl methyl cellulose for dispersion, then add the remaining purified water (at room temperature), stir until the solution is clear, and then add talcum powder and stir for 30 min for standby;

Coating: Put the plain pellets into the fluidized bed, set the fan speed at 1750-2000rpm, set the air inlet temperature at 40-50 ° C, which can be adjusted according to the actual situation. Preheat the pellets, control the material temperature at 37-39 ° C, and then start the atomization pressure at 0.13-0.18Mpa. Start the peristaltic pump to start spraying liquid. The spraying speed increases from slow to fast, and the peristaltic pump speed is controlled at 8-12rpm (pipe inner diameter is 5mm). Pay attention to monitoring the material temperature and fluidization state during coating, and the parameters can be adjusted according to the actual situation to prevent the pellet adhesion.

Sixth, enteric coating

Preparation of coating solution: Stir the weighed absolute ethanol into a vortex, slowly add 227.02g of hydroxypropyl methylcellulose acetate succinate for swelling, slowly add purified water (cold water), stir until the solution is clear, then add talcum powder, triethyl citrate and glyceride monostearate in turn, stir for 30min for standby;

Coating: Put the isolation coating pellets into the fluidized bed (Shenzhen Xinyite Technology Co., Ltd, The parameters can be adjusted according to the actual situation to prevent the pellet adhesion.

Seventh, capsule filling

Based on the content of enteric coated pellets, determine the capsule volume and then fill the capsule. Loading capacity: theoretical value ± 5%, loading capacity difference: ± 7.5%.

### 2. Stability inspection

Stability inspection method: under the conditions of 40 ± 2 ° C and 75 ± 5% RH, samples were taken in the month 0, 1, 2 and 3 to test the related substances, acid resistance and release rate. A specific detection method is as follows:

### 2.1 Related substances

Take an appropriate amount of enteric coated pellets prepared in this Example (about equivalent to 65mg of orbitazine fumarate), weigh it accurately and place in a 25ml volumetric flask, add an appropriate amount of diluent (DMSO-methanol, 1:4v/v) to dissolve orbitazine fumarate via ultrasound for 15min, allow it to cool, dilute with diluent to volume to the notch, shake up, centrifuge at 12000 rpm for 10min, and take the supernatant as the test solution. Accurately measure 1ml of the test solution and put into a 100ml volumetric flask, dilute with the diluent to the scale, shake up, and use as the control solution. In addition, take appropriate amount of the reference substance of orbitazine fumarate, the reference substance of impurity A, the reference substance of impurity C and triethyl citrate (TEC) respectively, and dilute them with diluent to obtain a mixed solution, each containing about 25µg of orbitazine fumarate, impurity A, impurity C and triethyl citrate per 1 mL.

According to the high performance liquid chromatography (Chinese Pharmacopoeia, 2015 Edition, Part IV, General Rule 0512), octadecyl silane bonded silica gel is used as the filler (recommended chromatographic column: YMC-PACK Pro C18,4.6mm × 100mm,3 µ m); take methanol acetonitrile (1:3) as mobile phase B, and take perchlorate buffer solution of pH 2.2 (weigh 7.0g of sodium perchlorate, add 1000ml of water to dissolve, adjust the pH to 2.2 with perchloric acid, and filter) - mobile phase B (82:18) as mobile phase A; Perform gradient elution as per Table 2 below; column temperature shall be subject to gradient control according to Table 3 below; The detection wavelength is 210 nm; the flow rate is 1.0 mL per minute.

**Table 2. Mobile Phase Gradient Details**

| Time (min) | Mobile phase A (%) | Mobile phase B (%) |
|---|---|---|
| 0 | 100 | 0 |
| 4 | 100 | 0 |
| 10 | 81 | 19 |
| 25 | 65 | 35 |
| 35 | 17 | 83 |
| 44 | 17 | 83 |
| 60 | 100 | 100 |

**Table 3. Details of Column Temperature Gradient**

| Time (Min) | Temperature of the left temperature control box | Temperature of the right temperature control box |
|---|---|---|
| 0 | 15°C | |
| 4.5 | 15°C | |
| 4.6 | 45°C | |
| 30 | 45°C | |
| 30.1 | 15°C | |
| 60 | 15°C | |

Take 3 µL of mixed solution to inject into the liquid chromatograph, record the chromatogram with the peak sequence as follow: fumaric acid, impurity A, triethyl citrate, orbitazine and impurity C. Take 3 µL of reference solution to inject into the liquid chromatograph, adjust the detection sensitivity, and make the peak height of orbitazine about 20%-30% of the full range. Accurately measure 3 µL of blank solution and 3 µL of test solution respectively to inject into the liquid chromatograph, and record the chromatogram. If there is an impurity peak in the chromatogram of the test solution, deduct the chromatographic peak of the blank solution and fumaric acid peak, triethyl citrate peak and impurity C peak in the mixed solution, and after multiplying with the correction factor, the peak area of impurity A (RRT is about 0.22, calculated by the method of correction factor (1.88)) shall not be greater than 0.5 times (0.5%) of the main peak area of the reference solution, The peak area of other single impurities shall not be greater than 0.2 times (0.20%) of the main peak area of the reference solution. Any peak in the chromatogram of the test solution smaller than 0.05 times (0.05%) of the main peak area of the reference solution can be ignored.

### 2.2 Extent of release

Take the enteric coated pellets prepared in this example, and detect according to the method for measuring dissolution and release (Chinese Pharmacopoeia, 2015 Edition, Part IV, first method of General Rule 0931, Method 2). Take 900 ml of hydrochloric acid solution (9 → 1000) as dissolution medium, with speed of 100 rpm, run under the rules, and after 120 minutes, the basket is immediately lift out of the liquid level and the hydrochloric acid solution discarded, and then 900ml of phosphate buffer solution (take 68.05g of potassium dihydrogen phosphate, add 152ml of 1mol/L sodium hydroxide, dilute to 10000ml with water, shake up, with pH of 6.0) was preheated to 37 °C and added, continue to operate under the rules. After 45 minutes, take the solution and filter it to become the test solution. In addition, accurately weigh 10 mg of orbitazine fumarate reference sample, place it in a 100ml volumetric flask, add ethanol for dissoluation, dilute to the scale, and shake up to become the reference solution. Take the test solution and the reference solution to be measured by the method according the concentration, and calculate the dissolution amount of each capsule.

### 2.3 Acid resistance

Take the enteric coated pellets prepared in this example as the test sample. According to the method for measuring dissolution and release (the first method of General Rule 0931), take 900 ml of hydrochloric acid solution (9 → 1000) as the dissolution medium, and the rotational speed is 100 rpm. Operate according to the rule. After 120 minutes, take out the test sample, wash out the hydrochloric acid solution on the surface with water, transfer an appropriate amount of absolute ethanol to a 100ml measuring flask, and then dissolve the orbitazine fumarate with ultrasound for 15 minutes. Placed to cool, dilute to the scale with anhydrous ethanol, shake up, filtered (or centrifuged), take 1ml of filtrate (or supernatant) into a 10ml volumetric flask, dilute to the scale with anhydrous ethanol, shake up to be used as the test solution. Determine according to the method according the concentration, and calculate the content of each capsule. The dissolution amount of each capsule is not greater than 10% of the labeled amount.

The stability test results are shown in Table 4.

**Table 4. Stability test results of the preparation**

| Stability test results | | | Notes |
|---|---|---|---|
| | 0 day | No impurities detected | |
| | Accelerate 1 month | Impurity C: 0.12% | Impurity C is 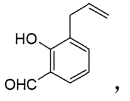 |
| | | Impurity A: ND | Impurity A is |
| Related substance | Accelerate 2 months | Impurity C: 0.14% | 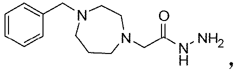 |
| | | Impurity A: ND | |
| | Accelerate 3 months | Impurity C: 0.19% | |
| | | Impurity A: ND | both are degradation impurities of orbitazine fumarate |
| Acid resistance (%) (in hydrochloric acid medium with pH=1.0 for 2h) | 0 day | 3.0 | Lower than 10% |
| | | 0.4 | |
| | | 1.0 | |
| | Accelerate 1 month | 1.2 | |
| | | 4.0 | |
| | | 1.7 | |
| | Accelerate 2 months | 4.6 | |
| | | 2.9 | |
| | | 3.3 | |
| | Accelerate 3 months | 2.9 | |
| | | -0.9 | |
| | | -0.2 | |
| 45min extent of release (%) (in hydrochloric acid medium with pH=1.0 for 2h, replace the phosphate medium basket method with pH=6.0 at 100rpm, n=6) | 0 day | 87.4±3.0 | Higher than 70% |
| | Accelerate 1 month | 85.5±0.9 | |
| | Accelerate 2 months | 85.4±2.0 | |
| | Accelerate 3 months | 82.0±1.6 | |

Conclusion: It can be seen from the data in the above table, after three months of accelerated experiment, 1) the measured impurities are only the degradation impurities 1 of orbitazine, and the concentration is very low, indicating that the enteric coated pellets have good stability and drug safety; 2) The acid resistance and release of enteric coated pellets capsule did not change significantly in the accelerated period of 3 months, which further explained the stability of the preparation.

### Example 2: Pharmacokinetic tests

Eight beagles were divided into two groups with four beagles in each group, using a two cycle crossover and self controlled experimental design. Each cycle, capsule T (the content is the enteric coated pellets of orbitazine fumarate prepared in Example 1, 100 mg) or capsule R (hydroxypropyl oridazine-β cyclodextrin inclusion complex solution) 10 mL (100 mg). Fast for 12h before administration, and control drinking water before administration and within 4h after administration. Take food 5 hours after administration with the wash out period of 7 days. (Note: Preparation method of drug R: appropriate amount of hydroxypropyl-β cyclodextrin is added into 100mL purified water for dissolution, and 1g of orbitazine fumarate API is added into water, stirred and dissolved to obtain 10 mg/mL inclusion complex, which is divided into 10 portions, 10 mL each).

Blood sample collection: 3mL to 5mL of blood was collected from forelimb veins prior to administration (0 h) and after administration at 0.25, 0.5, 0.75, 1, 1.25, 1.5, 2, 3, 4, 6, 8, 10, 12 and 24 h. The blood sample was centrifuged at 3000 r/min for 10 min within 15 min after collection, and the plasma was separated. The plasma was transferred to a clean 2mL EP tube to detect the content of orbitazine in the plasma.

The detection method of orbitazine in plasma is as follows:

| | | |
|---|---|---|
| Substance to be tested | Orbitazine | |
| Internal standard | Liranate | |
| Method of analysis | The concentration of orbitazine in dog plasma was determined by HPLC with liranate as the internal standard, following the validation protocol of the test and the company SOP of this invention. | |
| Equipment name | Manufacturer | Model |
| Liquid phase | Shimadzu | LC04 |
| Data processing system | Shimadzu | Lab-Solution |
| Analysis software | Lab-Solution software (Shimadzu, Japan) | |
| Model, specification, serial number and batch number of chromatographic column | Chromatographic column: Agilent 5TC-C₁₈ (2) (4.6×150 mm,5 µm) S.N.497562, P.N.588935-902 | |
| Mobile phase and flow rate | Acetonitrile: 10mMammonium acetate (83:13, v/v), flow rate: 0.45mL/min | |
| Column temperature | 35°C | |
| Biomatrix of the sample | Beagle plasma | |
| Anticoagulant | Heparin sodium | |
| Biomatrix source | Healthy beagles | |
| Sample pretreatment method | Acetonitrile protein precipitation | |
| Injection volume | 40 µL | |
| Quantitative index | Peak area ratio of target analyte to internal standard | |
| Regression equation | Linear fitting, equation expression, such as y=ax+b | |
| Weight coefficient | 1/X² | |
| Linear range | 50-4000ng/mL | |
| LLOQ | 50 ng/mL | |
| LOQ QC | 150 ng/mL | |
| MOQ QC | 900 ng/mL | |
| HOQ QC | 3000 ng/mL | |
| Residues in batch testing | Substance to be tested carryover 0.0%, internal standard carryover 1.9%. | |

Calculate the measured data of the concentration of oridazine in plasma at each time point after a single administration in beagle dogs, the average concentration (Mean) at each time point, and provide the concentration time curve (C-T curve), average C-T curve, and standard deviation of each time point of the C-T curve for each beagle dog after a single administration of orbitazine. The results are shown in Table 5 and Figure 1.

**Table 5: Pharmacokinetic parameters of beagle dogs after single oral administration of two preparations**

| Parameters | Orbitazineenteric coated pellets (Drug T) | Orbitzine hydroxypropyl-β Cyclodextrin inclusion complex (drug R) |
|---|---|---|
| AUC₀₋₁₂ₕ (ug/L*h) | 6665.781 | 3697.832 |
| AUC_{0-∞} (ug/L*h) | 7977.234 | 4182.582 |
| MRT₀₋₁₂ₕ (h) | 5.093 | 3.48 |
| MRT_{0-∞} (h) | 9.32 | 5.19 |
| t_{1/2} (h) | 6.06 | 3.66 |
| Tₘₐₓ (h) | 1.563 | 0.906 |
| Cₘₐₓ (ug/L) | 1288.408 | 1124.452 |

### Result analysis:

(1) The peak time of blood concentration of drug T in beagles (Tₘₐₓ=1.563h) was later than that of drug R (Tₘₐₓ=0.906). AUC_{0-∞}(7977.234ug/L*h) of drug T was significantly higher than that of drug R (4182.582µg/L*h), which shows that the enteric coated pellets capsule prepared in Example 1 of the invention has stable release in vivo and high bioavailability.
(2) Peak blood concentration of drug T in beagle dogs Cₘₐₓ(1288.408µg/L) has no significant difference with drug R. This data shows that the toxicity of the main drug does not increase while the bioavailability (effectiveness) of the enteric coated pellets of orbitazine is improved, and the drug safety is guaranteed.

### Example 3: Compatibility test of the enteric coating material

This example is to investigate the compatibility of the enteric coated material with orbitazine fumarate. The preparation provided by the invention is enteric coated pellets capsule, which is well absorbed in the duodenum, and the preparation needs to be rapidly released after entering the intestine. Stronger acidity of the enteric coating material, lower pH of dissolution, and better solubility in alkaline conditions, are more favorable to release. But at the same time, the stronger the acidity of the enteric coating material, the more unfavorable the stability of the enteric coated pellets of the invention is, and the greater the thickness of the isolation coating is required. Therefore, to ensure that the enteric coating material can be dissolved at a lower pH, the risk of affecting the stability of the preparation should be minimized.

In this example, the enteric coating material with dissolution pH as close as possible to the duodenal pH range are selected as candidate materials. Acrylic resin (Eudragit L100-55), HPMC-AS (LG), Obaday (enteric coated 91 series PVAP) and Hydroxypropylmethylcellulose phthalate HPMCP (HP-55), which can be dissolved at pH ≥ 5.5, meet the above requirements.

Test method: Weigh 2 portions of about 5mg enteric coating material and 1 portion of API orbitazine fumarate, place one portion of the enteric coating material in beaker ① and the other portion in beaker ②, add 100ml of each configured phosphoric acid buffer salt medium with pH=6.0 into two beakers at the same time, put the two beakers into a 37 °C water bath at the same time, stir at the same time, add API to beaker ② after the enteric coating material is dissolved, and observe the phenomenon. Take cup ② sample to detect the API concentration, and calculate the proportion of its dissolution and interaction amount in the total amount. The results are shown in Table 6.

**Table 6. Compatibility test results of enteric coating materials and orbitazine fumarate**

| Group | Enteric coated material | Phenomenon in beaker ① | Phenomenon in beaker ② | API measured amt after mixing (mg) | Ratio of interaction amt in total amt (%) |
|---|---|---|---|---|---|
| 1 | Eudragit L100-55 | rapid dissolvation, clear solution | White precipitate | 1.43 | 75.26 |
| 2 | HPMC-AS (LG) | Dissolution rate is slow, and most of them are dissolved at 45 min, but not all of them are dissolved | API dissolution, HPMC-AS is not completely dissolved, the phenomenon is the same as that of beaker ① | 5.16 | 7.03 |
| 3 | Obaday (PVAP) | Can be completely dissolved in 15 min | API dissolves, but then flocs are formed and suspended | 0.91 | 81.81 |
| 4 | HPMCP (HP-55) | Dissolution rate is slow, about half of solution is dissolved in 45 min, but not completely dissolved | API dissolved rapidly, but HPMCP did not dissolve completely at 45 min | 4.03 | 18.75 |
| Note: From the solubility of API, 5mg API can be completely dissolved in 100ml release medium, and the proportion of interaction amount in the total amount (%) = (API sample weight - API measured amount after mixing)/API sample weight×100 | | | | | |

### Result analysis:

The complete dissolution in beaker ① of group 1 indicates that 5mg of Eudragit L100-55 can be completely dissolved in 100ml of release medium, but precipitation occurs in beaker ②, and the amount of orbitazine fumarate detected in the solution is very small, which can be reasonably inferred that there is an interaction between them and the effect is strong. In the same way, it can be seen that groups 2, 3 and 4 also have different degrees of interaction, and the order of strength is as follows: Eubalde (PVAP) > Acrylic resin (Eudragit L100-55) > HPMCP (HP-55) > HPMC-AS (LG). From the perspective of stability and preparation release risk, HPMC-AS is applicable to enteric coated pellets of the invention, followed by HPMCP.

### Example 4: Screening of alcohol concentration of wetting agent

The concentration of alcohol of wetting agent was designed to vary between 50% and 95% to prepare pellets. Taking roundness and disintegration time as the research object, the optimal ethanol concentration was selected. Feed the materials according to the formula in Table 7. Use 75%, 60% and 50% ethanol to manually prepare soft materials. Use an extrusion plate with a hole diameter of 0.9 mm. The extrusion speed is 30 rpm. The extruded materials are rounded in the rotary table. Adjust the rounding parameters according to the material conditions to prepare pellets. The prepared pellets are dried in a fluidized bed (air inlet temperature: material temperature: 35 °C-45 °C) to prepare plain pellets. Observe the roundness of the pellets and determine the disintegration time. The results are shown in Table 7.

**Table 7. Alcohol concentration screening results**

| Component | Manufacturer | Model | Prescription dosage (mg/pellet) | | | | | |
|---|---|---|---|---|---|---|---|---|
| | | | J20161010-2 | | J20161011-2 | | J20161011-1 | |
| | | | mg/ pellet | Ratio/ % | mg/ pellet | Ratio/ % | mg/ pellet | Ratio/ % |
| API | Dongguan Chang'an East Sunshine Co., Ltd. | - | 100.00 | 45.0 | 100.00 | 45.0 | 100.00 | 45.0 |
| Microcrystalli ne cellulose | DFE Phama | PH 301 | 51.11 | 23.0 | 51.11 | 23.0 | 51.11 | 23.0 |
| PVP k30 | Ashland | K30 | 6.67 | 3.0 | 6.67 | 3.0 | 6.67 | 3.0 |
| Twain 20 | Xilong Chemicals | - | 11.11 | 5.0 | 11.11 | 5.0 | 11.11 | 5.0 |
| Cross-linked povidone | Ashland | XL-10 | 22.22 | 10.0 | 22.22 | 10.0 | 22.22 | 10.0 |
| Low substituted hydroxylpropyl cellulose | Anhui Shanhe Auxiliary Materials Co., Ltd. | SH-LH 21 | 31.11 | 14.0 | 31.11 | 14.0 | 31.11 | 14.0 |
| Sum | | | 222.22 | 100 | 222.22 | 100 | 222.22 | 100 |
| Batch size (pellets) | | | 450 | | 450 | | 450 | |
| Wetting agent | | | 75% Ethanol | | 60% Ethanol | | 50% Ethanol | |
| Roundness of plain pellets (visual inspection) | | | Poor, more columnar and dumbbell shaped | | Rounder | | Rounder | |
| Disintegration time (s) | | | 10 | | 30 | | 60 | |

The results showed that when the ethanol concentration was 60%, the pellets had good roundness and fast disintegration (30s). On the premise of ensuring the roundness, 60% ethanol concentration is selected as the wetting agent to ensure rapid disintegration and rapid release of the plain pellets.

### Example 5: Screening of disintegrants

Cross linked povidone (XL-10), cross-linked sodium carboxymethyl cellulose and sodium carboxymethyl starch were selected as the objects of study. The conventional maximum dosage was used, and they were used together with low substituted hydroxypropyl cellulose to keep the dosage of other excipients in the prescription unchanged. The dosage of diluent was fine tuned to make the drug loading of pellets consistent. 60% ethanol is used as the wetting agent to manually prepare soft materials. The aperture of the extrusion plate is 0.9 mm, and the extrusion speed is 30 rpm. The extruded materials are rounded in the rotary table. The rounding parameters are adjusted according to the shape of the materials to prepare pellets. The prepared pellets are dried in a fluidized bed (the inlet air temperature is set at 45 °C, and the material temperature is controlled at 35 °C-45 °C) to prepare plain pellets. Then HPMC is used to coat the isolation coating. The speed of the fluidized bed inlet fan is 1200 rpm, the speed of the peristaltic pump is 6-12 rpm, the calculated liquid spraying speed is about 0.5-1.4g/min, the atomization pressure is controlled to be about 0.04Mpa, and the coating liquid is fully atomized. The material stability is controlled to be 35-45 °C, and the weight increase is 3%. Then use HPMC-AS for enteric coating. The speed of the fluidized bed inlet fan is 1200rpm, the speed of the peristaltic pump is 6-12rpm, the calculated liquid spraying speed is about 1.1g-1.7g/min, the atomization pressure is controlled to be about 0.04Mpa, and the coating liquid is fully atomized. The material temperature is controlled to be 35°C-45°C, and the weight gain is 18% (theoretical feeding). The coating formula and process are the same. Test the release and acid resistance of the pellets after filling the capsules, and the results are shown in Table 8.

**Table 8. Screening results of disintegrants**

| Component | Manufacturer | Model | | Prescription dosage (mg/pellet) | | |
|---|---|---|---|---|---|---|
| | | | | C20161011 | C20161108 | C20170114-1 |
| Plain pellets | | | | | | |
| API | Dongguan Chang'an East Sunshine Co., Ltd. | - | | 100.00 | 100.00 | 100.00 |
| Microcrystalline cellulose | DFE Phama | PH301 | | 51.11 | 55.56 | 60.00 |
| Pvp k30 | Ashland | K30 | | 6.67 | 6.67 | 6.67 |
| Twain 20 | Xilong Chemicals | - | | 11.11 | 11.11 | 11.11 |
| Cross-linked povidone (XL-10) | Ashland | XL-10 | | 22.22 | - | - |
| Carboxymethyl starch sodium (Libeng) | Anhui Shanhe Auxiliary Materials Co., Ltd. | High expansion type | | - | 17.78 | - |
| Cross linked hydroxymethyl cellulose sodium | Anhui Shanhe Accessories Co., Ltd. | N/A | | - | - | 13.33 |
| Low substituted hydroxypropyl cellulose | Anhui Shanhe Accessories Co., Ltd. | | SH-LH21 | 31.11 | 31.11 | 31.11 |
| Sum (mg) | | | | 222.22 | 222.22 | 222.22 |
| Batch size (pellets) | | | | 450 | 450 | 450 |

| Isolation coating | | | | | | |
|---|---|---|---|---|---|---|
| HPMC | Anhui Shanhe Accessories Co., Ltd. | | E3 | 5.33 | 5.33 | 5.33 |
| Talc powder | Aladdin | | 325 mesh | 1.33 | 1.33 | 1.33 |

| Enteric coating | | | | | | |
|---|---|---|---|---|---|---|
| HPMC-AS | Asahi Kasei, Japan | | LG | 28.56 | 26.09 | 26.09 |
| Talc powder | Aladdin | | 325 mesh | 9.52 | 8.70 | 8.70 |
| TEC | Aladdin | | E3 | 7.25 | 6.64 | 6.64 |
| Twain 80 | Wenzhou Qingming Chemicals | | N/A | 1.13 | 0.92 | N/A |
| Glyceryl monostearate | Yuanye Biotechnology Co., Ltd. | | 352 mesh | 1.13 | 0.92 | 0.92 |
| Acid resistance/% (2h in hydrochloric acid medium with pH=1.0) | | | | 1.5 | 0.8 | 4.3 |
| | | | | 2.9 | 0.1 | |
| | | | | 1.8 | 0.0 | 4.1 |
| | | | | / | 0.5 | |
| | | | | 3.5 | -1.4 | 4.6 |
| | | | | 6.7 | 1.1 | |
| Release/% (2h in hydrochloric acid medium with pH=1.0, 100 rpm in phosphate medium basket method with pH=6.0, n=3) | | 10min | | 3.5 | 4.2 | 1.0 |
| | | 15min | | 6.9 | 22.7 | 6.5 |
| | | 30min | | 26.0 | 53.4 | 36.4 |
| | | 45min | | 41.8 | 65.0 | 57.1 |
| | | 60min | | 53.8 | 71.3 | 67.6 |
| | | 90min | | 67.7 | 77.0 | 78.7 |

The results showed that the prescription proportion was the maximum conventional dosage (10% cross-linked povidone (22.22 mg per unit prescription), 8% sodium carboxymethyl starch (17.78 mg per unit prescription), and 6% cross-linked sodium carboxymethyl cellulose (13.33 mg per unit prescription). The release effect of pellets from high to low was sodium carboxymethyl starch (Libeng), sodium carboxymethyl cellulose (Libeng), and cross-linked povidone (XL-10). When sodium carboxymethyl starch (Libeng) was used as the disintegrant, its swelling power was the largest, Enteric coated pellets disintegrate best and release fastest. Therefore, sodium carboxymethyl starch (Libeng) is selected as the disintegrant of this product. Therefore, the C20161108 batch prescription was determined as the target prescription of plain pellets.

### Example 9: Effect of weight gain of isolation coating on release and stability of enteric coated pellets

The isolation coating can prevent the API from direct contact and interacting with the enteric coating material HPMC-AS, which affects the release and stability of the preparation. API and HPMC-AS can only be prevented from direct contact or contact after penetration after the isolation coat completely wraps the drug loaded plain pellets and reaches a certain thickness. If the barrier is incomplete or too thin, it will cause API and HPMC-AS in the release medium to interact with each other under local high concentration, and slow down the release. The thicker the isolation coat (the greater the weight gain), the weaker this interaction is, and the HPMC itself also has the characteristics of non-ionic surface activity, which is beneficial to the release of the preparation. In addition, the acidity of the enteric coating material is an unstable factor (degradation) of the API, so the weight gain of the isolation coating is beneficial to the stability of the preparation related substances.

The grain size of the plain pellets is about 0.6-1.2mm, and the surface area is large. It requires a certain coating weight increase to completely wrap the surface of the plain pellets. According to the diameter of the pellets, when the coating weight increase is 6.8%, the surface of the pellets can be completely covered by the isolation clothing. The weight gain of this example is designed to be 7%, 15% and 19%, and the coating effect is investigated. Hydroxypropyl methyl cellulose is selected as the isolation coating material, talcum powder is used as the anti-adhesive (the dosage ratio is 4:1), the speed of the fluidized bed inlet fan is adjusted according to different batches, 1200-1800 rpm, the speed of the peristaltic pump is 6-8, the calculated liquid spraying speed is about 0.7-1.5 g/min, the atomization pressure is about 0.04-0.12 Mpa, and the coating liquid is fully atomized, and the material stability is controlled at 35 °C-45 °C. Hydroxypropylmethylcellulose acetate succinate (HPMC-AS) was selected as the enteric coating material, talcum powder as the anti-adhesive, triethyl citrate as the plasticizer, and monostearin as the anti-static material, with the dosage ratio (11.4:3.8:2.8:0.4). The speed of the fluidized bed air inlet fan was 1200-1400rpm, the speed of the peristaltic pump was 6-12, and the spraying speed was 0.8-1.3g/min. The coating liquid was fully atomized by controlling the atomization pressure of 0.04-0.06Mpa. The actual material temperature was controlled at 35 °C-42 °C, Weight gain 18%. The enteric coated pellets were tested for release and acid resistance after being filled with capsules, and the results are shown in Table 9.

**Table 9. Effect of weight gain of isolation coating on release of enteric coated pellets**

| Component | Manufacturer | | Model | Prescription dosage (mg/pellet) | | |
|---|---|---|---|---|---|---|
| | | | | C20170406 | C20170419-1 | C20170419-2 |
| API | Dongguan Chang'an East Sunshine Co., Ltd. | | - | 100.00 | 100.00 | 100.00 |
| Microcrystall ine cellulose | DFE Phama | | PH301 | 55.55 | 55.55 | 55.55 |
| PVP k30 | Ashland | | K30 | 6.67 | 6.67 | 6.67 |
| Twain 20 | Xilong Chemicals | | - | 11.11 | 11.11 | 11.11 |
| Carboxymeth yl starch sodium (Libeng) | Anhui Shanhe Auxiliary Materials Co., Ltd. | | High expansi on type | 17.78 | 17.78 | 17.78 |
| Low substituted hydroxyprop yl cellulose | Anhui Shanhe Accessories Co., Ltd. | | SH-LH 21 | 31.11 | 31.11 | 31.11 |
| Sum (mg) | | | | 222.22 | 222.22 | 222.22 |
| Batch size (pellets) | | | | 450 | 450 | 450 |

| Isolation coating | | | | | | |
|---|---|---|---|---|---|---|
| HPMC | Anhui Shanhe Accessories Co., Ltd. | | E3 | 12.44 | 26.67 | 33.78 |
| Talc powder | Aladdin | | 325 mesh | 3.11 | 6.67 | 8.45 |
| Coating weight gain (%) | | | | 7.0 (Actual weight gain 6.5) | 15.0 (Actual weight gain 14.1) | 19 (Actual weight gain 19.5) |

| Enteric coating | | | | | | |
|---|---|---|---|---|---|---|
| HPMC-AS | Shinyue, Japan | | LG | 26.09 | 29.13 | 30.15 |
| Talc powder | Aladdin | | 325 mesh | 8.70 | 9.71 | 10.05 |
| TEC | Aladdin | | N/A | 6.64 | 7.16 | 7.40 |
| Glyceryl monostearate | Yuanye Biotechnology Co., Ltd. | | N/A | 0.92 | 1.02 | 1.06 |
| Coating weight gain (%) | | | | 18.0 (Actual weight gain 18.0) | 18.0 (Actual weight gain 16.9) | 18.0 (Actual weight gain 16.4) |
| Acid resistance (%) (2h in hydrochloric acid medium with pH=1.0, n=3/6) | | | | 1.5 | -1.1 | -5.4 |
| | | | | 2.0 | -0.5 | |
| | | | | 3.0 | -0.6 | -2.4 |
| | | | | 2.7 | 3.0 | |
| | | | | 3.3 | 0.4 | 4.1 |
| | | | | 2.1 | 1.0 | |
| Release ± SD (%) (2h in hydrochloric acid medium with pH=1.0, 100rpm in phosphate medium basket method with pH=6.0, n=3/6) | | 10min | | 37.0±2.3 | 37.9±4.5 | 49.8±4.6 |
| | | 15min | | 64.3±2.5 | 67.9±7.7 | 76.1±5.1 |
| | | 30min | | 80.3±2.1 | 86.0±4.3 | 89.0±1.4 |
| | | 45min | | 83.7±2.1 | 87.4±3.0 | 89.2±1.9 |
| | | 60min | | 86.5±1.3 | 88.3±2.4 | 89.5±1.9 |
| | | 90min | | 89.0±0.7 | 88.7±2.3 | 90.3±1.5 |

Obitazine fumarate is unstable under acidic conditions, the enteric coating material is acidic, and the weight gain of the isolation coating affects the integrity and thickness of the isolation coating film. In this example, the weight gain of the isolation coating was investigated, and the isolation coating weight gain that can effectively block the orbitazine fumarate and the enteric coated material was screened. The main items to be investigated were concentration, related substances, acid resistance and release. The results are shown in Table 10.

**Table 10. Effect of weight gain of isolation coating on release of enteric coated pellets**

| Batch number | | | C20170406 | C20170419-1 | C20170419-2 |
|---|---|---|---|---|---|
| Isolation coating weight gain (%) | | | 7.0 (Actual weight gain 6.5) | 15.0 (Actual weight gain 14.1) | 19 (Actual weight gain 19.5) |
| Enteric coating weight gain (%) | | | 18.0 (Actual weight gain 18.0) | 18.0 (Actual weight gain 16.9) | 18.0 (Actual weight gain 16.4) |
| Stability inspection (acceleration 40°C/R H75%) | Acid resistance (%) (2h in hydrochloric | 0 mon | 1.5 | -1.1 | -5.4 |
| | | | 2.0 | -0.5 | |
| | | | 3.0 | -0.6 | -2.4 |
| | | | 2.7 | 3.0 | |
| | | | 3.3 | 0.4 | -4.1 |
| | | | 2.1 | 1.0 | |
| | | 15 d | Not tested | Not tested | Not tested |
| | acid medium with pH=1.0, n=3/6) | 1 mon | Not tested | 1.2 | -2.9 |
| | | | | 4.0 | -1.2 |
| | | | | 1.7 | -1.8 |
| | | 2 mon | Not tested | 4.6 | 1.1 |
| | | | | 2.9 | -0.2 |
| | | | | 3.3 | 0.4 |
| | | 3 mon | Not tested | 2.9 | 1.7 |
| | | | | -0.9 | 10.1 |
| | | | | -0.2 | 3.3 |
| | | | | 0.8 | 0.0 |
| | | | | 1.2 | 3.5 |
| | | | | 1.4 | 6.8 |
| | 45minre lease±S D (%) | 0 mon | 83.7±2.1 | 87.4±3.0 | 89.2±1.9 |
| | | 15 d | 77.5±2.5 | 86.8+0.8 | 90.9±1.7 |
| | | 1 mon | 67.0±4.1 | 85.5±0.9 | 89.9±1.2 |
| | | 2 mon | Not tested | 85.4±2.0 | 90.1±1.4 |
| | | 3 mon | Not tested | 82.0+1.6 | 84.3±3.2 |
| | Related substances % | 0 d | Impurity A: ND, Impurity C: ND, Other unknown impurities ND, Total impurities except C ND | Impurity A: ND, Impurity C: ND, Other unknown impurities ND, Total impurities except C ND | Impurity A: ND, Impurity C: ND, Other unknown impurities ND, Total impurities except C ND |
| | | 1 mon | No further detection of related substances due to slow release | Impurity A: ND, Impurity C: 0.12, Other unknown impurities ND, Total impurities except C ND | Impurity A: ND, Impurity C: 0.10, Other unknown impurities ND, Total impurities except C ND |
| | | 2 mon | Not tested | Impurity A: ND, Impurity C: 0.14, Other unknown impurities ND, Total impurities except C ND | Impurity A: ND, Impurity C: 0.12, Other unknown impurities ND, Total impurities except C ND |
| | | 3 mon | Not tested | Impurity A: ND, Impurity C: 0.19, Other unknown impurities ND, Total impurities except C ND | Impurity A: ND, Impurity C: 0.19, Other unknown impurities ND, Total impurities except C ND |

The results show the following:
(1) The release of enteric coated pellets in accelerated experiment slowed down significantly when the weight of isolation coat coating increased by 6.5%. The main reason is that when the weight gain is 6.5%, the isolation coating can completely wrap the plain pellets, so is qualified at day 0, but the thickness of the isolation coating is not enough under the lower weight gain. During the placement process, the molecular movement may cause the API or the enteric coating material to penetrate the isolation coating and contact each other, and the interaction may lead to a decrease in the release. When the weight gain of the isolation coating increased to 14.1% and 19.5%, the acid resistance and release of the enteric coated pellets capsules did not change significantly in the accelerated 3 months, and were relatively stable. It indicates that although the previous experiment showed that the day 0 release of the preparation reached the development goal when the coating weight increased by 6.5%, the coating weight increase of the isolation coat should not be less than 14.1% from the perspective of stability.
(2) Both 14.1% and 19.5% of the weight increase of the isolation coat coating and enteric coated pellets capsule related substances had an increase in impurity C, which was not detected in day 0. The accelerated growth of impurity C in 3 months was 0.19%, with the same growth rate, meeting the stability requirements. Therefore, from the perspective of related substances, 14.1% (15% for feeding) or more of the isolation clothing can effectively block the enteric coating and protect the orbitazine fumarate.

### Example 10: Investigation on weight gain of enteric coating

When the roundness and pellet size distribution of the plain pellets are the same, the greater the coating weight, the thicker the coating layer, and the stronger the acid resistance; The thicker the enteric coated membrane, the slower it will dissolve, and the release of enteric coated pellets will become slower. In order to ensure that the acid resistance meets the requirement, the coating weight gain should be as low as possible to ensure both the release and the stability of the preparation. In this example, three experiments were designed, in which the weight gain of enteric coating was 18%, 22% and 26% respectively, to coat the isolation coating pellets prepared by the same prescription and process. The speed of the fluidized bed inlet fan is 1200-1400rpm, the speed of the peristaltic pump is 6-12rpm, the liquid spraying speed is about 0.8-1.2g/min, the atomization pressure is controlled to be about 0.03-0.04Mpa, and the coating liquid is fully atomized. The actual material temperature is controlled to be 35 °C-40 °C. The acid resistance and release of enteric coated pellets capsules were tested. See Table 11 for the results.

**Table 11. Effect of weight gain of enteric coating on acid resistance and release of the preparation**

| Name | | Specification | | Prescription (mg) | | |
|---|---|---|---|---|---|---|
| | | | | C20170406 | C20170412-1 | C20170412-2 |
| Isolation pellets | | - | | 228.89 | 228.89 | 228.89 |
| HPMC-AS | | LG | | 26.09 | 31.82 | 37.54 |
| Talc powder | | 325 mesh | | 8.70 | 10.60 | 12.59 |
| TEC | | - | | 6.64 | 7.94 | 9.38 |
| Glyceryl monostearate | | - | | 0.92 | 1.37 | 1.37 |
| Enteric coating weight gain (%) | | | | 18 (Actual weight gain 18.0) | 22 (Actual weight gain 19.5) | 26 (Actual weight gain 23.4) |
| Test items | Acid resistance (%) (2h in hydrochloric acid medium with pH=1.0) | | | 1.5 | -- | -- |
| | | | | 2.0 | -- | -- |
| | | | | 3.0 | -- | -- |
| | | | | 2.7 | -- | -- |
| | | | | 3.3 | -- | -- |
| | | | | 2.1 | -- | -- |
| | Release ±SD(%) | | 10min | 37.0±2.3 | 8.4±1.0 | 6.4±1.3 |
| | | | 15min | 64.3±2.5 | 29.0±3.4 | 28.9±3.0 |
| | | | 30min | 80.3±2.1 | 61.1±3.2 | 63.8±3.2 |
| | | | 45min | 83.7±2.1 | 69.5±2.8 | 72.0±1.8 |
| | | | 60min | 86.5±1.3 | 72.8±3.0 | 74.5±2.1 |

| | | | | | | |
|---|---|---|---|---|---|---|
| Note: Since the acid resistance of enteric coating can be guaranteed when the weight is increased by 18%, the acid resistance of 22% and 26% are not tested. | | | | | | |

The results show that:
(1) The weight of enteric coating was increased by 18%, which could ensure that the release of orbitazine fumarate enteric coated pellets capsule reached the development goal;
(2) When the weight gain of enteric coated pellets increased from 18% to 26%, the release rate of enteric coated pellets capsules slowed down, indicating that the enteric coating should not be too high; otherwise it is not conducive to the release of the preparation. With comprehensive consideration of acid resistance, 18% enteric coating is the most preferrable.

Although particular embodiments and examples are described herein in detail, the above description is provided by way of example for purposes of illustration only, and is not intended to be limiting with respect to the scope of the invention. In particular, it is contemplated by the inventor that various substitutions, alterations, and modifications may be made to the invention without departing from the scope of the invention as claimed.

## Claims

1. An enteric coated pellet, comprising: (a) a pellet core containing orbitazine fumarate, (b) an isolation layer, and (c) an enteric layer, wherein the enteric layer comprises an enteric coating material, which is hydroxypropyl methylcellulose acetate succinate, or hydroxypropyl methylcellulose phthalate.

2. The enteric coated pellet according to claim 1, wherein the pellet core further contains a diluent, disintegrant and cosolvent.

3. The enteric coated pellet according to claim 2, wherein the diluent is selected from one or more of microcrystalline cellulose, lactose, and pregelatinized starch.

4. The enteric coated pellet according to claim 2, the disintegrant is selected from one or more of sodium carboxymethyl starch, low substituted hydroxypropyl cellulose, cross-linked sodium carboxymethyl cellulose, and cross-linked povidone.

5. The enteric coated pellet according to claim 2, wherein the cosolvent is selected from one or more of polyvinylpyrrolidone, Tween-20, Tween-60, Tween-80, and sodium dodecyl sulfate.

6. The enteric coated pellet according to claim 4, wherein the disintegrant is:
sodium carboxymethyl starch,
cross-linked sodium carboxymethyl cellulose,
cross-linked povidone,
combination of low substituted hydroxypropyl cellulose and sodium carboxymethyl starch,
combination of low substituted hydroxypropyl cellulose and cross-linked sodium carboxymethyl cellulose, or
combination of low substituted hydroxypropyl cellulose and cross-linked povidone.

7. The enteric coated pellet according to claim 6, wherein:
mass ratio of the low substituted hydroxypropyl cellulose to the sodium carboxymethyl starch is 0.1-3.5:1,
mass ratio of the low substituted hydroxypropyl cellulose to the cross-linked sodium carboxymethyl cellulose is 0.1-3.5:1,
mass ratio of the low substituted hydroxypropyl cellulose to the cross-linked povidone is 0.1-3.5:1.

8. The enteric coated pellet according to claim 1, wherein the pellet core comprises, by weight:
orbitazine fumarate, 10-30 portions;
microcrystalline cellulose, 4-12 portions;
sodium carboxymethyl starch, 0-5 portions;
low substituted hydroxypropyl cellulose, 2-8 portions;
Tween, 0-4 portions;
polyvinylpyrrolidone, 0-3 portions.

9. The enteric coated pellet according to claim 1, wherein the isolation layer comprises isolation materials.

10. The enteric coated pellet according to claim 9, wherein the isolation layer comprises also an anti-adhesive, and the isolation material is one or both of hydroxypropyl methyl cellulose and hydroxypropyl cellulose.

11. The enteric coated pellet according to claim 1, wherein the enteric layer also comprises a plasticizer and/or an anti-adhesive.

12. The enteric coated pellet according to claim 1, comprising, by weight:
orbitazine fumarate, 10-30 portions;
microcrystalline cellulose, 4-12 portions;
sodium carboxymethyl starch, 0-5 portions;
low substituted hydroxypropyl cellulose, 2-8 portions;
Tween, 0-4 portions;
polyvinylpyrrolidone, 0-3 portions;
hydroxypropyl methyl cellulose, 1-5 portions;
hydroxypropyl methylcellulose acetate succinate, 1-5 portions;
triethyl citrate, 0-3 portions;
glyceryl monostearin, 0-0.5 portions; and
talcum powder, 0-5 portions.

13. The enteric coated pellet according to claim 1, wherein mass percentages of a) the pellet core containing orbitazine fumarate, b) the isolation layer, and c) the enteric coated layer are as follows:
a) the pellet core 60%-78%;
b) the isolation layer 10%-20%, and
c) the enteric layer 12%-20%.

14. A method of preparation of the enteric coated pellet according to claim 1, the method comprising steps of:
a) mixing orbitazine fumarate with diluent and disintegrant to obtain a premix;
b) mixing the premix with a cosolvent to prepare a soft material, wherein the cosolvent is obtained by mixing the cosolvent with a 50%-70% ethanol aqueous solution;
c) the soft material is extruded, rounded, pelleted and dried;
d) wrapping with an isolation layer;
e) wrapping with an enteric layer.

15. The enteric coated pellet according to any one of claims 1-13 for use in the treatment of tumors.

## Patentansprüche

1. Ein Pellet mit enterischer Schutzhülle, das Folgendes umfasst: (a) einen Pelletkern, der Orbitazinfumarat, (b) eine Isolierungsschicht und (c) eine enterische Schutzhülle, wobei die enterische Schutzhülle ein enterisches Beschichtungsmaterial umfasst, das Hydroxypropyl Methylzellulosenacetatsuccinat ist oder Hydroxypropylmethylzellulose Phafhalat.

2. Das Pellet mit enterischer Schutzhülle gemäß Anspruch 1, wobei der Pelletkern weiter ein Verdünnungsmittel, ein Zersetzungsmittel und ein Hilfslösungsmittel enthält.

3. Das Pellet mit enterischer Schutzhülle gemäß Anspruch 2, wobei das Verdünnungsmittel ausgewählt wird aus einer oder mehreren folgender Substanzen: mikrokristalline Zellulose, Laktose und vorgelatinierte Stärke.

4. Das Pellet mit enterischer Schutzhülle gemäß Anspruch 2, wobei das Zersetzungsmittel aus einer oder mehreren folgender Substanzen ausgewählt wird: Natrium Carboxymethylstärke, niedrig substituierte Hydrozypropylzellolose, vernetzte Natriumcarboxymethylzellolose und vernetztes Povidon.

5. Das Pellet mit enterischer Schutzhülle gemäß Anspruch 2, bei dem das Hilfslösungsmittel ausgewählt wird aus einer oder mehreren Substanzen: Polyvinylpyrrolidon, Tween-20, Tween-60, Tween-80 und Natriumdodecylsulfat.

6. Das Pellet mit enterischer Schutzhülle gemäß Anspruch 4, bei dem das Zersetzungsmittel Folgendes ist:
Natriumcarboxymethylstärke,
vernetzte Natriumcarboxymethylzellulose,
vernetztes Povidon,
eine Kombination von niedrig substituierter Hydroxypropylzellulose und Natriumcarboxymethylstärke,
eine Kombination von niedrig substituierter Hydroxypropylcellulose und vernetztem Natrium Carboxymethylcellulose, oder
eine Kombination von niedrig substituierter Hydroxypropylzellulose und vernetztem Povidon.

7. Das Pellet mit enterischer Schutzhülle gemäß Anspruch 6, Wobei:
das Massenverhältnis der niedrig substituierten Hydroxypropylzellulose mit Bezug auf die Natriumcarboxymethylstärke folgendes ist: 0,1-3,5: 1,
das Massenverhältnis der niedrig substituierten Hydrozxypropylzellulose mit Bezug auf die verlenetzte Natriumcarboxylmethylzellolose 0,1-3,5 : 1 ist,
das Massenverhältnis der niedrig substituierten Hydroxypropylzellulose mit Bezug auf das vernetzte Povidon 0,1-3,5 : 1 ist.

8. Das Pellet mit enterischer Schutzhülle gemäß Anspruch 1, wobei der Pelletkern gewichtsmässig Folgendes umfasst
Orbitazinfumarat, 10-30 Anteile;
Mikrokristalline Zellulose, 4-12 Anteile;
Natrium Carboxymethylstärke, 0-5 Anteile
Niedrig substituierte Hydroxypropylzellulose, 2-8 Anteile
Tween, 0-4 Anteile;
Polyvinylpirrolidon, 0-3 Anteile.

9. Das Pellet mit enterischer Schutzhülle gemäß Anspruch 1, wobei die Isolierschicht Isolierungsmaterial umfasst.

10. Das Pellet mit enterischer Schutzhülle gemäß Anspruch 9, wobei die Isolierschicht auch ein Antiadhäsive umfasst und das Isolierungsmaterial entweder nur eine oder beide folgende Substanzen umfasst: Hydroxypropyl Methylzellolose und Hydroxypropyl Zellulose.

11. Das Pellet mit enterischer Schutzhülle gemäß Anspruch 1, wobei die enterische Schutzhülle auch einen Weichmacher oder ein Antiadhäsiv umfasst.

12. Das Pellet mit enterischer Schutzhülle gemäß Anspruch 1, das pro Gewicht Folgendes umfasst:
Orbitazinfumarat, 10-30 Anteile;
Mikrokristalline Zellulose, 4-12 Anteile;
Natrium Carboxymethylstärke 0-5 Anteile
Niedrig substituierte Hydroxypropyl Zellulose, 2-8 Anteile
Tween, 0-4 Anteile;
Polyvinylpirrolidon, 0-3 Anteile;
Hydroxypropyl Methylzellolose, 1-5 Anteile;
Hydroxypropyl Methylzellulose Azetatsuccinate, 1-5 Anteile
Triethylzitrat, 0-3 Anteile;
Glyzerilmonostearin, 0-0,5 Anteile, und
Talcumpuder, 0-5 Anteile.

13. Das Pellet mit enterischer Schutzhülle gemäß Anspruch 1, wobei die Massenprozente von a) dem Pelletkern mit Orbitazinfumaratinhalt, b) der Isolierungsschicht und c) die Schicht mit Schutzhülle folgende sind:
a) Der Pelletkern 60%-78%;
b) Die Isolierungsschicht 10%-20%, und
c) Die enterische Schutzhülle 12%-20%.

14. Eine Methode für die Herstellung des Pellets mit enterischer Schutzhülle gemäßAnspruch 1, wobei die Methode folgende Schritte umfasst:
a) Mischen des Orbitazinefumarats mit einem Verdünner und einem Desintegrator zwecks Erhalts einer Vormischung;
b) Mischen der Vormischung mit einem Kosolvenz zwecks Zubereitung eines Weichmaterials, wobei das Kosolvenz durch Mischen des Kosolvenz mit einer 50%-70%igen wässrigen Ethanol-Lösung erhalten wird;
c) Das Weichmaterial wird extrudiert, gerundet, pelletiert und getrocknet;
d) Umhüllen mit einer Isolierungsschicht;
e) Umhüllen mit einer enterischen Schutzhülle.

15. Das enterisch beschichtete Pellet gemäß irgendeinem der Ansprüche 1-13 für die Anwendung bei Tumoren.

## Revendications

1. Un pellet à enrobage entérique, comprenant : (a) un noyau de pellet contenant du fumarate d'orbitazine, (b) une couche d'isolation, et (c) une couche entérique, dans laquelle la couche entérique comprend un matériau d'enrobage entérique, qui est soit le succinate d'acétate d'hydroxypropylméthylcellulose, soit le phtalate d'hydroxypropylméthylcellulose.

2. Le pellet à enrobage entérique selon la revendication 1, dans lequel le noyau du pellet contient en outre un diluant, un agent désintégrant et un co-solvant.

3. Le pellet à enrobage entérique selon la revendication 2, dans lequel le diluant est choisi parmi une ou plusieurs des substances suivantes : cellulose microcristalline, lactose et amidon prégélatinisé.

4. Le pellet à enrobage entérique selon la revendication 2, dans lequel le désintégrant est choisi parmi un ou plusieurs des substances suivantes : amidon carboxyméthylique sodique, hydroxypropylcellulose faiblement substituée, carboxyméthylcellulose sodique réticulée, et povidone réticulée.

5. Le pellet à enrobage entérique selon la revendication 2, dans lequel le co-solvant est choisi parmi une ou plusieurs des substances suivantes : polyvinylpyrrolidone, Tween-20, Tween-60, Tween-80, et dodécylsulfate de sodium.

6. Le pellet à enrobage entérique selon la revendication 4, dans lequel l'agent désintégrant est choisi parmi un des produits suivants :
carboxyméthylamidon sodique,
carboxyméthylcellulose sodique réticulée,
povidone réticulée,
une combinaison d'hydroxypropylcellulose faiblement substituée et de carboxyméthylamidon sodique,
une combinaison d'hydroxypropylcellulose faiblement substituée et de carboxyméthylcellulose sodique réticulée, ou
une combinaison d'hydroxypropylcellulose faiblement substituée et de povidone réticulée.

7. Le pellet à enrobage entérique selon la revendication 6, dans lequel :
Le rapport massique de l'hydroxypropylcellulose faiblement substituée par rapport au carboxyméthylamidon sodique est de 0,1-3,5 : 1,
Le rapport massique de l'hydroxypropylcellulose faiblement substituée par rapport à la carboxyméthylcellulose sodique réticulée est de 0,1-3,5 : 1,
Le rapport massique de l'hydroxypropylcellulose faiblement substituée par rapport à la povidone réticulée est de 0,1-3,5 : 1.

8. Le pellet à enrobage entérique selon la revendication 1, dans lequel le noyau du pellet comprend, en poids, les substances suivantes :
fumarate d'orbitazine, 10-30 portions ;
cellulose microcristalline, 4-12 portions ;
carboxyméthylamidon sodique, 0-5 portions ;
hydroxypropylcellulose faiblement substituée, 2-8 portions ;
Tween, 0-4 portions ;
polyvinylpyrrolidone, 0-3 portions.

9. Le pellet à enrobage entérique selon la revendication 1, dans lequel la couche d'isolation comprend des matériaux d'isolation.

10. Le pellet à enrobage entérique selon la revendication 9, où la couche d'isolation contient également un agent anti-adhésif et où le matériau d'isolation comprend soit l'un, soit les deux des substances suivantes : hydroxypropylméthylcellulose et hydroxypropylcellulose.

11. Le pellet à enrobage entérique selon la revendication 1, dans lequel la couche entérique comprend également un plastifiant et/ou un anti-adhésif.

12. Le pellet à enrobage entérique selon la revendication 1, comprenant, en poids, les substances suivantes :
fumarate d'orbitazine, 10-30 portions ;
cellulose microcristalline, 4-12 portions ;
carboxyméthylamidon sodique, 0-5 portions ;
hydroxypropylcellulose faiblement substituée, 2-8 portions ;
Tween, 0-4 portions ;
polyvinylpyrrolidone, 0-3 portions ;
hydroxypropylméthylcellulose, 1-5 portions ;
succinate d'acétate d'hydroxypropylméthylcellulose, 1-5 portions ;
citrate de triéthyle, 0-3 portions ;
monostéarate de glycéryle, 0-0. 5 portions ; et
talc, 0-5 portions.

13. Le pellet à enrobage entérique selon la revendication 1, où les pourcentages massiques de : a) le noyau du pellet contenant du fumarate d'orbitazine, b) la couche d'isolation et c) la couche d'enrobage entérique sont les suivants :
a) le noyau du pellet 60%-78% ;
b) la couche d'isolation 10%-20%, et
c) la couche d'enrobage entérique 12%-20%.

14. Procédé de préparation du pellet à enrobage entérique selon la revendication 1, comprenant les étapes suivantes :
a) mélange du fumarate d'orbitazine avec un diluant et un agent désintégrant pour obtenir un prémélange ;
b) mélange du prémélange avec un co-solvant pour préparer une matière molle, le co-solvant étant obtenu par mélange du co-solvant avec une solution aqueuse d'éthanol à 50-70% ;
c) extrusion, arrondissement, transformation en pellets et séchage de la matière molle ;
d) enrobage avec une couche d'isolation ;
e) enrobage avec une couche entérique.

15. Le pellet à enrobage entérique selon une quelconque des revendications 1 à 13, destiné à être utilisé dans le traitement des tumeurs.
